# EUROPÄISCHE PATENTSCHRIFT

(11) **EP 1 800 615 B1**
(45) Veröffentlichungstag und Bekanntmachung des Hinweises auf die Patenterteilung: **12.12.2007**
(21) Anmeldenummer: 05028290.4
(22) Anmeldetag: 23.12.2005
(51) Int. Cl.: A61B 17/80

(54) **Knochenplatte**
Bone plate
Plaque de fixation d'os

(43) Veröffentlichungstag der Anmeldung: 27.06.2007
(73) Patentinhaber: aap Implantate AG, 12099 Berlin (DE)
(72) Erfinder: Ahrens, Uwe, 10179 Berlin (DE); Bakhshi, Majid, 12053 Berlin (DE); Fischer, Dr. Hans-Joachim, 12277 Berlin (DE)
(74) Vertreter: von Eichel-Streiber, Caspar

(56) Entgegenhaltungen:
- EP-B- 0 760 632
- CH-A5- 650 915
- DE-A1- 4 343 117
- US-A1- 2004 087 951

## Beschreibung

Die Erfindung betrifft eine Knochenplatte.

Knochenplatten sind beispielsweise bekannt aus der CH 462375, der WO 2000/53111, der WO 2001/54601 oder der EP 0760632 B1.

Die Ausgestaltung der Löcher ist bei diesem Stand der Technik so, dass sie aus der Kombination von zwei sich überschneidenden Löchern bestehen mit unterschiedlichen Durchmessern und zu einem Durchgangsloch kombiniert sind und jede Seite des Durchgangslochs mit seinem festen Durchmesser seine eigene Funktionalität aufweist und auch getrennt voneinander betrachtet werden muss.

So ist es beispielsweise nicht möglich, auf der gewindeanteiligen Seite des Durchgangsloches eine Standard-Kortikalisschraube einzubringen, sondern nur eine Schraube mit Kopfgewinde.

Die andere Seite des Durchgangsloches, die keinen Gewindeanteil aufweist, ist nur für die Einbringung einer Standard-Kortikalisschraube geeignet, die auch mit Kompression und Winkelvariabilität appliziert werden kann.

Somit könnte man die Löcher auch völlig voneinander trennen da sie keine funktionellen Schnittpunkte aufweisen.

Dieser Nachteil zieht sich dann auch durch die gesamte Anwendung hin, denn beim Einbringen einer Standard-Kortikalisschraube muss auch darauf geachtet werden, dass man sich auch immer auf der richtigen Seite des Durchgangsloches befindet.

Aus der US 2004/0087951 A1 ist eine Knochenschraube bekannt, die an ihrem Schraubenkopf in Richtung der Schraubenlängsachse gesehen übereinander angeordnet einen Gewindeabschnitt und glattwandige Abschnitte aufweist. Diese glattwandigen Abschnitte dienen der gelenkigen Aufnahme der Schraube in entsprechenden Führungen.

Ausgehend hiervon ist es die Aufgabe der Erfindung, die Einsatzmöglichkeiten einer Knochenplatte zu verbessern und deren Flexibilität in der Handhabung zu erhöhen.

Gelöst wird diese Aufgabe durch eine Knochenplatte mit den Merkmalen des Anspruchs 1. Vorteilhafte Weiterbildungen sind in den abhängigen Ansprüchen 2 bis 8 angegeben. In Anspruch 9 schließlich ist ein System aus einer erfindungsgemäßen Knochenplatte und wenigstens einer dazu passenden Knochenschraube angegeben, das insgesamt als ein Fixierungssystem angesehen werden kann.

Erfindungswesentlich ist für die neuartige Knochenplatte, dass in einer Richtung quer zu der Ebene ihrer Oberseite gesehen gleichermaßen übereinander angeordnet in einem Teilabschnitt sowohl ein Gewinde als auch eine glattwandige Haltestruktur angeordnet sind. Durch das Zusammenwirken von Gewinde und glattwandiger Haltestruktur kann eine mit entsprechenden Gegenstrukturen ausgebildete Knochenschraube besonders effektiv lagefixiert in der Knochenplatte gehalten werden. Durch das Zusammenwirken des Gewindes in dem Langloch mit einem entsprechend ausgebildeten Gegengewinde am Kopf bzw. Hals der Knochenschraube wird die Knochenschraube mit einer korrespondierend zu der Haltestruktur ausgebildeten Gegenstruktur bzw. Anlagestruktur fest in diese Haltestruktur gepresst und so sicher fixiert.

Dabei ist es grundsätzlich egal, ob von der Oberseite der Platte her gesehen die Haltestruktur zuerst und im unteren Bereich der Knochenplatte das Gewinde angeordnet ist oder umgekehrt. Ebenso gut können wechselnd und jeweils unterbrochen durch wenigstens einen Abschnitt mit einer Haltestruktur mehrere Gewindeabschnitte in der Knochenplatte vorgesehen sein. Bevorzugt wird ein Ausführungsbeispiel, bei dem der Gewindeabschnitt nahe der Oberseite der Knochenplatte liegt und eine Haltestruktur nahe der Unterseite der Knochenplatte (vgl. Anspruch 4).

Der mit Gewinde und Haltestruktur versehene Abschnitt des Langloches ist erfindungsgemäß auf einen Teilabschnitt des Langloches beschränkt, er liegt vorzugsweise an einer Schmalseite des Langloches (vgl. Anspruch 8).

Insbesondere ist in einer vorteilhaften Weiterbildung vorgesehen, dass das Gewinde in der Ebene der Oberfläche gesehen maximal 180° eines kreisförmigen Umfanges eines mit diesem in Eingriff zu bringenden Schraubenkopfes bzw. eines -halses einer Knochenschraube umgreift. Diese Ausgestaltung bewirkt, dass eine Knochenschraube entlang der Längsachse des Langloches frei verschiebbar ist, somit also sowohl zum Aufbringen einer auf die Frakturstelle hingerichteten Anpresskraft als auch zum winkelfesten Fixieren der Platte verwendet werden kann.

Derzeit wird als Haltestruktur eine teilsphärische Fläche bevorzugt. In dieser kann eine entsprechend sphärisch bzw. teilsphärisch ausgebildete Oberfläche des Kopfes bzw. Hal ses der Knochenschraube festgelegt werden. Prinzipiell ist es auch möglich, andere Formen der Oberflächen der Haltestrukturen einzusetzen, beispielsweise konisch.

Ein wie gemäß einer vorteilhaften Weiterbildung nach Anspruch 5 vorgesehener Einstich im Übergangsbereich zwischen Gewinde und Haltestruktur erleichtert eine Überführung des Schraubenkopfes bzw. -halses, genauer des mit einem Gewinde versehenen Bereiches desselben, in den mit dem Gewinde versehenen Abschnitt des Langloches, insbesondere bei einem Verschieben der Knochenschraube entlang des Langloches.

In einer Ausgestaltung gemäß Anspruch 6 ist vorgesehen, dass das Langloch an der Oberseite der Knochenplatte eine umlaufende Führungsstruktur aufweist. Diese dient der Führung des Kopfes oder Halses einer Knochenschraube. Diese Führungsstruktur kann beispielsweise ein umlaufender Rand mit teilkreisförmig ausgebildetem Querschnitt sein, in dem eine entsprechend sphärisch bzw. teilsphärisch ausgebildete Gegenstruktur am Kopf bzw. Hals einer Knochenschraube gleiten kann. Gemäß Anspruch 6 definiert die Führungsstruktur eine Führungsebene. Diese Führungsebene ist eine solche Ebene, innerhalb derer ein gedachter Punkt am Schraubenkopf gleitet, wenn die Schraube entlang der Führungsstruktur verschoben wird. Ferner wird diese Führungsebene von einer Längsachse des Gewindes, dies ist im Sinne der Erfindung die Achse, um die ein in das Gewinde einzuschraubendes Gegengewinde bei einem Einschraubvorgang rotiert, um einen von 90° verschiebenden Winkel geschnitten. Diese Art der Verkippung zwischen Gewinde und Führungsebene bewirkt, dass auch bei einer in der Ebene der Oberfläche gelegenen Umschlingung von maximal 180° das Gewinde respektive die Haltestruktur dem Schraubkopf bzw. -hals einen sicheren Halt verleiht, da die Verkippung zu einer effektiv weitergehenden Umschlingung zumindest in einem Teilbereich des Gewindes führt. Dieser Effekt wird durch eine aufgrund der Verkippung ausgelöste "Klemm-" bzw. "Verkeilwirkung" erzielt.

Ferner wird bevorzugt, dass die Führungsebene um einen Winkel zwischen 0° und 90° gegenüber der Plattenebene geneigt ist (Anspruch 7). Hier wird ferner eine Ausführungsvariante bevorzugt, bei welcher das Gewinde bzw. die Haltestruktur in einem Abschnitt des Langloches geordnet sind, in dem die Führungsebene am tiefsten, d.h. am weitesten von der Oberseite der Knochenplatte entfernt, liegt (vgl. Anspruch 8).

Unter Langloch wird im Sinne dieser Erfindung nicht lediglich ein durch Verschieben eines Kreises entlang einer geraden Achse hervorgerufenes Lochbild verstanden, sondern grundsätzlich jegliche Öffnungsform, die in einer ersten Erstreckungsrichtung länger ist als in einer zweiten Erstreckungsrichtung und die durchgehend, d.h. ohne in das Loch hineinragende "Barrieren" verläuft. Somit kann ein Langloch im Sinne der Erfindung auch "sich dreieckig verjüngend" ausgebildet sein und oval.

Mit der Erfindung wird schließlich noch ein System bestehend aus einer wie oben beschriebenen Knochenplatte und einer Knochenschraube angegeben. Die Knochenschraube dieses Systems hat erfindungsgemäß einen Schraubenkopf bzw. Schraubenhals, an welchem übereinander geordnet und zu den entsprechenden Gegenstrukturen in der Knochenplatte passend arrangiert eine Gewindestruktur und eine komplementär zu der Haltestruktur des Langloches ausgebildete Anlagestruktur aufweist.

Die Knochenplatte kann mit einer solchen, speziell ausgebildeten Knochenschraube zugleich winkelstabil fixiert und gespannt werden. Es soll hier jedoch betont werden, dass die erfindungsgemäße Knochenplatte auch mit Standardknochenschrauben bzw. an solche Standardknochenschrauben angelehnten Knochenschrauben fixiert werden kann, die insbesondere weder ein Gewinde noch eine Anlagestruktur an ihrem Schraubenkopf bzw. -hals aufweisen.

Insgesamt wird bevorzugt, dass bei einer Ausgestaltung des Langloches mit der Führungsstruktur ein Abschnitt einer systemzugehörigen, erfindungsgemäß ausgebildeten Knochenschraube (beispielsweise das Gewinde) in seiner Außenkontur entsprechend dem Querschnitt der Führungsstruktur ausgebildet ist, so dass auch eine erfindungsgemäße Knochenschraube in der Führungsstruktur geführt werden kann. Damit kann auch eine ansonsten zum winkelstabilen Fixieren der Knochenplatte ausgebildete Knochenschraube zum "Spannen" der Knochenplatte Verwendung finden.

Die erfindungsgemäße Knochenplatte bietet - auch zusammen mit der systemzugehörigen Knochenschraube - eine große Vielzahl von Einsatzmöglichkeiten, die sie zum Fixieren von unterschiedlichsten Frakturen geeignet macht.

Hierfür wird insbesondere bevorzugt, dass sämtliche Öffnungen in der Knochenplatte als Langlöcher mit den erfindungsgemäßen Eigenschaften ausgebildet sind.

Weitere Vorteile und Merkmale der Erfindung ergeben sich aus der nachfolgenden Beschreibung eines Ausführungsbeispieles anhand der beigefügten Figuren. Dabei zeigen:
- Fig. 1: ein Ausführungsbeispiel für eine erfindungsgemäße Knochenplatte in einer dreidimensionalen Ansicht,
- Fig. 2: die Knochenplatte aus Figur 1 in einer Ansicht von oben,
- Fig. 3: in einem vergrößerten Ausschnitt die Unterseite der Knochenplatte aus Fig. 1,
- Fig. 4: in einer vergrößerten Darstellung die Unterseite einer alternativen Ausgestaltung einer erfindungsgemäßen Knochenplatte,

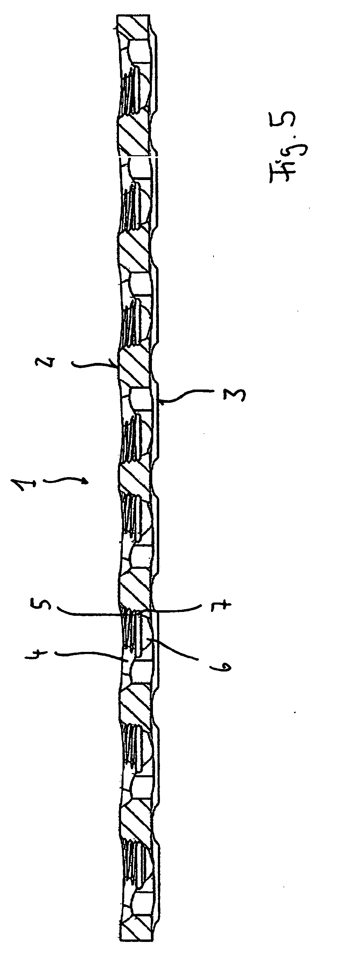

- Fig. 5: einen Querschnitt durch eine Knochenplatte aus Fig. 1 entlang ihrer Mittellängsebene,

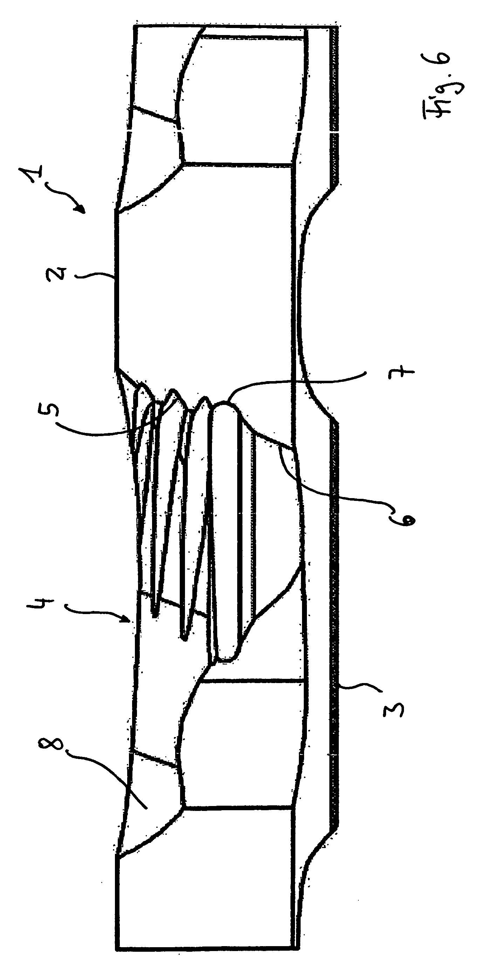

- Fig. 6: in vergrößerter Darstellung einen Querschnitt entsprechend Fig. 5 mit nur einem dargestellten Langloch,
- Fig. 7: eine Darstellung wie in Fig. 6 mit zur Veranschaulichung eingetragenem Verlauf der Führungsstruktur,
- Fig. 8: eine Ansicht einer Standardknochenschraube,
- Fig. 9: eine mit Fig. 8 vergleichbare Ansicht einer erfindungsgemäßen Knochenschraube zum Zusammenwirken mit dem Gewinde und der Haltestruktur in der erfindungsgemäßen Knochenplatte,
- Fig. 10: in einer schematischen Darstellung eine in ein Langloch der erfindungsgemäßen Knochenplatte eingesetzte Standardknochenschraube,
- Fig. 11: eine ähnliche Darstellung wie Fig. 10 mit der Standardknochenschraube in eine anderen Position,
- Fig. 12: eine der Fig. 10 vergleichbare Darstellung mit einer Knochenschraube gemäß Fig. 9 anstelle der Standardknochenschraube,
- Fig. 13: eine in dem Gewinde und der Haltestruktur festgelegte Knochenschraube gemäß Fig. 9,
- Fig. 14: eine Ansicht der erfindungsgemäßen Knochenplatte mit für das Bohren eines Schraubenloches in einem Knochen aufgesetzter Bohrhülse, und
- Fig. 15: eine ähnliche Ansicht wie Fig. 14 jedoch mit einer alternativen Variante einer Bohrhülse.

In den Figuren ist schematisch ein Ausführungsbeispiel einer erfindungsgemäßen Knochenplatte gezeigt und allgemein mit 1 bezeichnet. Das gezeigte Ausführungsbeispiel ist keinesfalls maßstabsgerecht, sondern dient lediglich einer prinzipiellen Erläuterung.

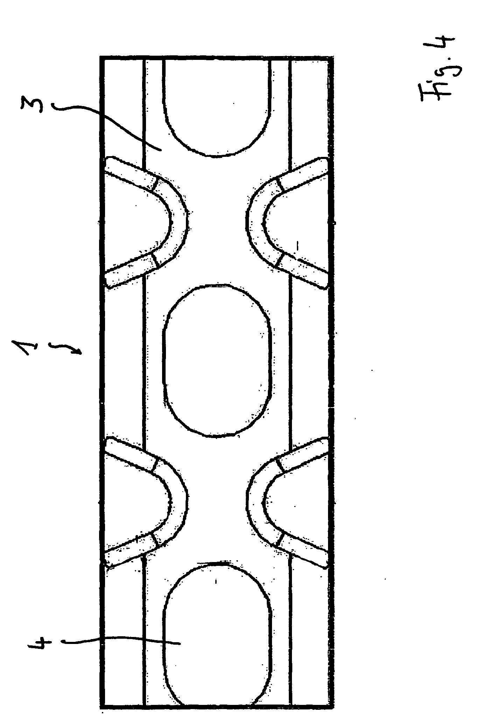

Die erfindungsgemäße Knochenplatte 1 hat eine Oberseite und eine Unterseite 3, welche zur Auflage auf den zu fixierenden Knochen dient. Zwischen Oberseite und Unterseite sind durchgehende Öffnungen in Form von Langlöchern 4 in der Knochenplatte eingebracht. Die Langlöcher 4 sind durchgehend, d.h. ohne etwaige nach innen reichende Vorsprünge oder ähnliche Hindernisse ausgebildet. Die erfindungsgemäße Knochenplatte 1 weist in diesem Ausführungsbeispiel insgesamt acht Langlöcher 4 auf, ohne jedoch auf diese Anzahl beschränkt zu sein. Die Langlöcher 4 sind in dem bevorzugten Ausführungsbeispiel sich zu einer der Längsseiten hin verjüngend ausgebildet. Sie können jedoch, wie beispielsweise in einer alternativen Ausgestaltung gemäß Fig. 4 gezeigt, auch gerade verlaufen und der Form entsprechen, wie sie durch Verschieben eines Kreises entlang einer vorgegebenen Strecke entsteht. Selbstverständlich sind auch alle anderen denkbaren Formen von Langlöchern möglich, solange diese durchgehend, d.h. in ihrem inneren hindernisfrei gestaltet sind.

An einer Stirnseite eines jeden Langloches sind ein Gewinde 5 sowie eine Haltestruktur 6 gebildet. Das Gewinde 5 und die Haltestruktur 6 liegen in einer Richtung quer zu der Plattenebene übereinander geordnet, wobei in diesem Ausführungsbeispiel das Gewinde 5 oben (der Oberseite 2 der Knochenplatte 1 zugewandt) und die Haltestruktur 6 unten (der Unterseite 3 der Knochenplatte 1 zugewandt) angeordnet sind. Im Übergangsbereich zwischen Gewinde 5 und Haltestruktur 6 ist ein Einstich 7 eingebracht. Die Haltestruktur 6 ist durch einen glattwandigen, teilsphärischen Wandabschnitt des Langloches gebildet.

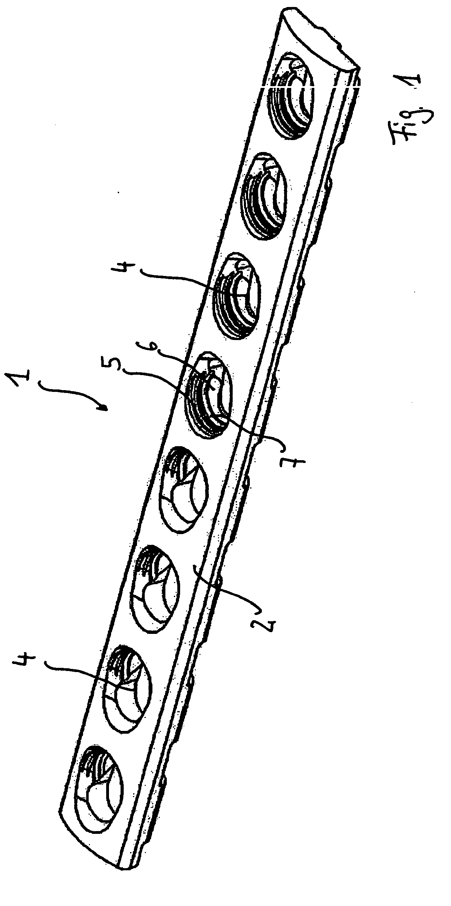

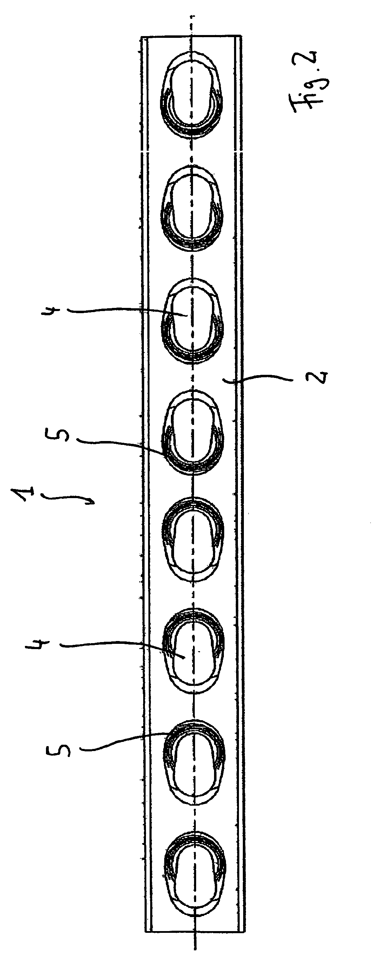

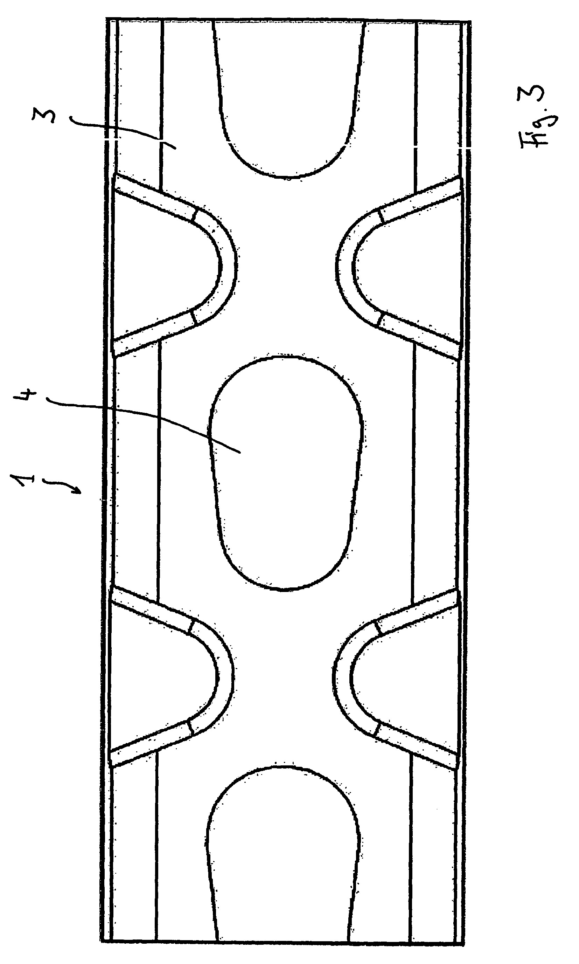

Wie insbesondere in den Figuren 1, 2 und 3 zu erkennen ist, sind bei dem gezeigten Ausführungsbeispiel die Gewinde 5 bzw. Haltestrukturen 6 der Langlöcher 4 an Schmalseiten der letztgenannten angeordnet, und zwar an den verjüngten Schmalseiten, die jeweils in Richtung der Plattenmitte ausgerichtet sind. Hierbei verlaufen die längeren Längsachsen der Langlöcher 4 entlang einer Plattenlängsachse.

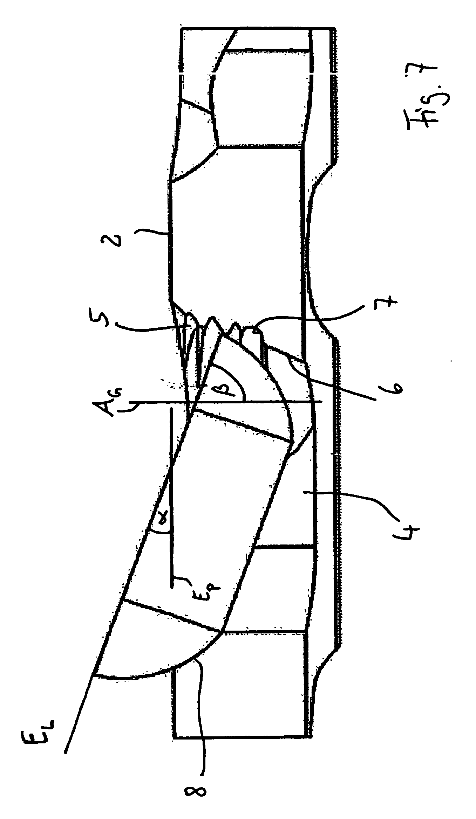

Das Langloch 4 enthält an seiner der Oberseite 2 der Knochenplatte 1 zugewandten Öffnung eine umlaufende Führungsstruktur 8. Diese ist durch einen Rand mit teilkreisförmigem Querschnitt gebildet, der in das Langloch 4 beispielsweise durch einen spanenden Bearbeitungsschritt (z.B. Fräsen) eingebracht wird. In Fig. 7 ist hierzu zur Veranschaulichung ein "badewannenartiger" Verlauf dieses Randes, also der Führungsstruktur 8 eingezeichnet. Hier ist auch besonders gut zu erkennen, dass die Führungsstruktur 8 um einen Winkel α zur durch den Verlauf der Oberseite 2 der Knochenplatte 1 festgelegten Verlauf der Plattenebene E_{P} geneigt ist. Gleichermaßen ist die Führungsebene E_{L} gegenüber einer Gewindelängsachse A_{G} ebenfalls geneigt, und zwar um einen Winkel β.

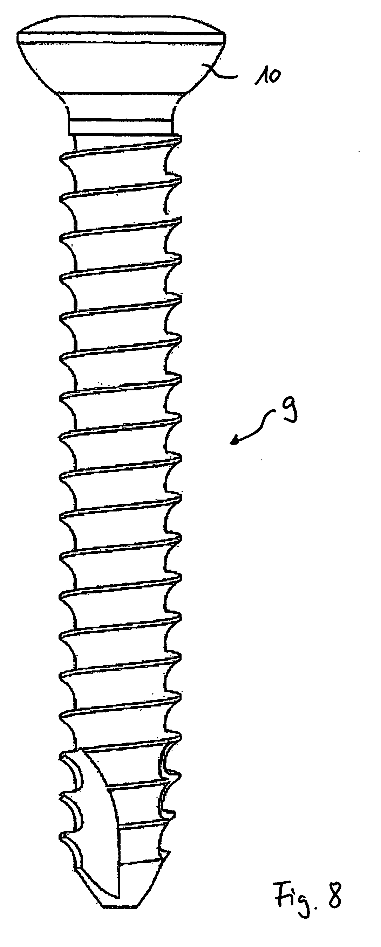

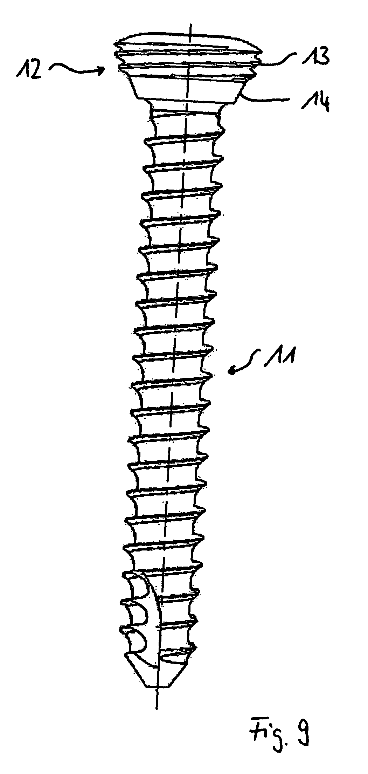

In den Figuren 8 und 9 sind eine Standard-Knochenschraube 9 (Fig. 8) mit einem glatt und an der Unterseite sphärisch geformten Schraubenkopf 10 bzw. eine speziell für das Zusammenwirken mit der erfindungsgemäßen Knochenplatte 1 konzipierte Knochenschraube 11 (Fig. 9) mit einem Schraubenkopf 12 gezeigt. Der Schraubenkopf 12 enthält an einem oberen Abschnitt ein in diesem Ausführungsbeispiel sphärisch gestaltetes Gewinde 13 und in Richtung der Längsachse der Schraube gesehen darunter einen Abschnitt mit einer Anlagestruktur 14. Diese Anlagestruktur 14 ist glattwandig gebildet mit sphärischen bzw. teilsphärischen Verlauf, und sie ist komplementär zu der Form der Haltestruktur 6 der Knochenplatte 1.

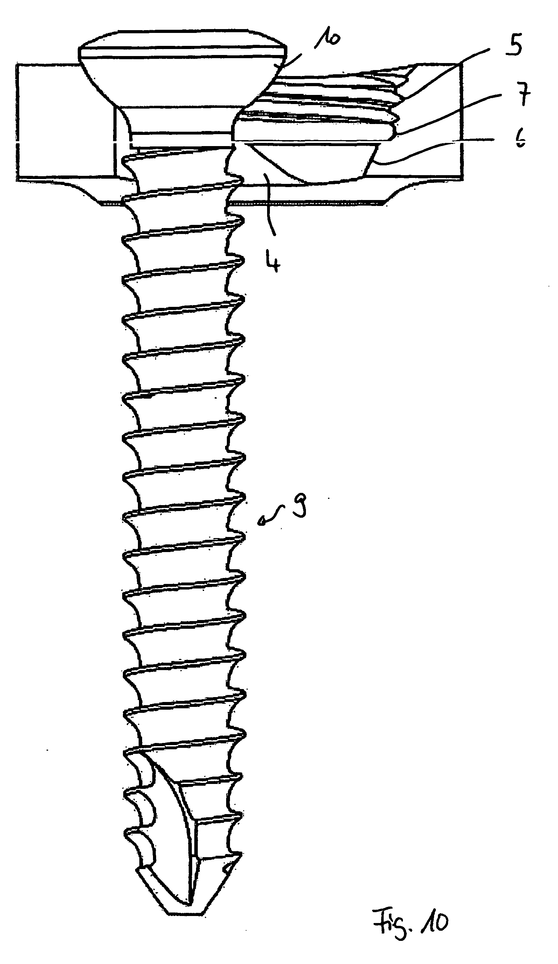

In den Figuren 10 bis 13 ist das Zusammenwirken zwischen einer Knochenschraube 9 der herkömmlichen Art bzw. einer Knochenschraube 11 der erfindungsgemäß angepassten Art und der erfindungsgemäßen Knochenplatte 1 gezeigt. So soll anhand dieser Figuren auch die Funktion der erfindungsgemäßen Knochenplatte 1 und ihrer Elemente nun erläutert werden.

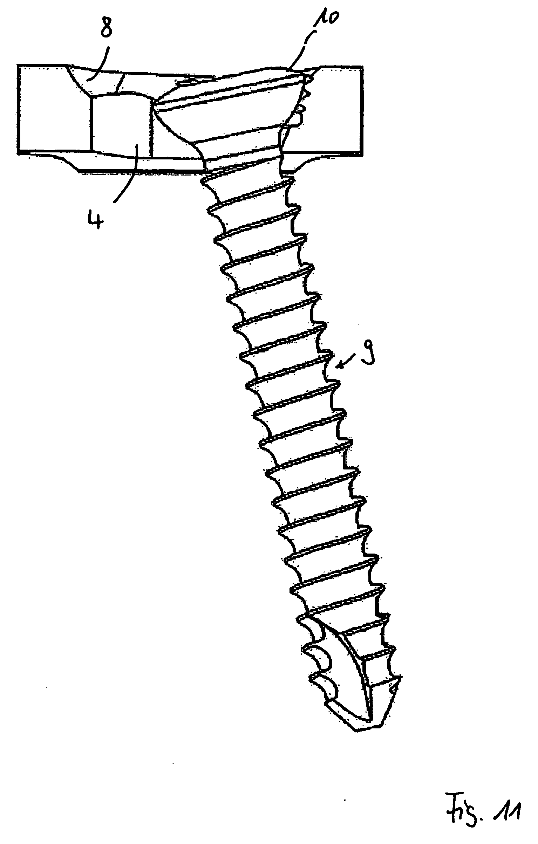

In den Figuren 10 und 11 ist in zwei unterschiedlichen Positionen eine in das Langloch 4 der Knochenplatte 1 eingesetzte, herkömmliche Knochenschraube 9 dargestellt. Diese Knochenschraube liegt mit der sphärisch glatt geformten Unterseite des Schraubenkopfes 10 auf der komplementär geformten Führungsstruktur 8 des Langloches 4 auf und kann so der Führungsebene E_{L} folgend entlang der Längsachse des Langloches 4 verschoben werden. Durch die Schiefstellung der Führungsebene E_{L} wird beim Einschrauben der Knochenschraube 9 eine Kompressionswirkung erzielt, indem die Knochenplatte 1 in eine Richtung gedrückt bzw. ein damit zu fixierender Knochen in die andere Richtung gezogen wird. Wie in Fig. 11 zu erkennen, sind die ins Innere des Langloches 4 weisenden Zahnungen des Gewindes 5 so geformt, dass sie ebenfalls eine komplementär zu der konisch geformten Unterseite des Schraubenkopfes 10 geformte Führungsfläche bilden. Diese bildet somit Bestandteil der Führungsstruktur 8. An dieser Führungsfläche kann die herkömmliche Knochenschraube 9 gegenüber der lotrechten (entsprechend der Richtung der Gewindeachse A_{G}) verkippt werden. Dies gibt eine Vielzahl von Möglichkeiten, die Knochenschraube 9 im Knochen festzulegen. Diese Verkippung ist nicht nur in einer Richtung entlang der Längsachse des Langloches hier möglich, sondern auch in Richtungen quer dazu, so dass sich letztlich ein Bereich von im wesentlichen 360° ergibt, in dem die Schraube gegenüber der Gewindeachse A_{G} gekippt werden kann.

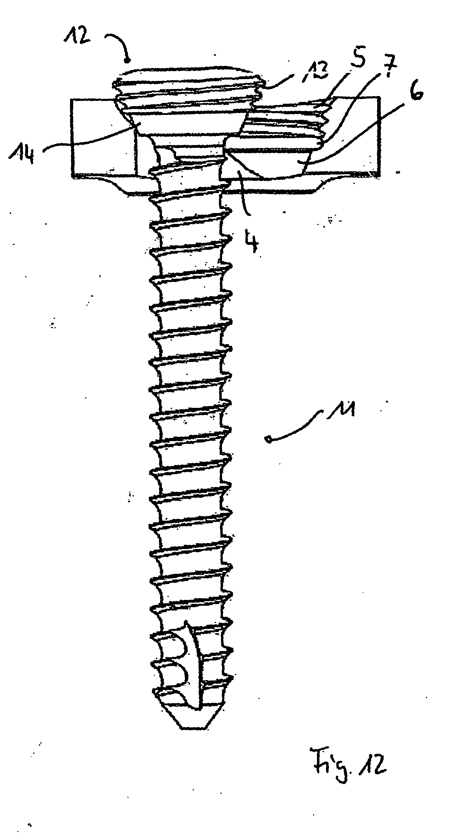

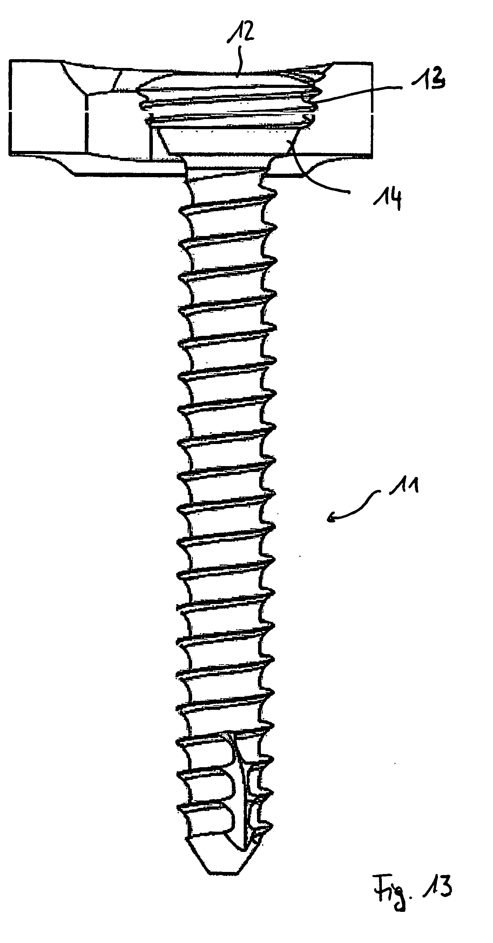

Eine solche herkömmliche Knochenschraube 9 kann mit der erfindungsgemäßen Knochenplatte 1 verwendet werden, um eine Kompressionswirkung zu erzielen. Nicht geeignet ist diese Knochenschraube allerdings, um eine winkelstabile Festlegung in Knochenplatte 1 und Knochen zu erzielen. Hierzu kann die erfindungsgemäß an die Knochenplatte angepasste Knochenschraube 11 verwendet werden, wie nachfolgend anhand der Figuren 12 und 13 beschrieben.

Der mit dem Gewinde 13 versehene Abschnitt des Schraubenkopfes 12 weist eine Außenkontur (eine Einhüllende der Gewindezüge) auf, die ebenfalls sphärisch und komplementär zu der Kontur der Führungsstruktur 8 ist. Auf diese Weise kann die Knochenschraube 11 genauso in der Führungsstruktur 8 geführt werden, wie die Knochenschraube 9. So kann auch die Knochenschraube 11 zunächst zum Erzielen einer Kompressionswirkung eingesetzt werden. Die Besonderheit der Knochenschraube 11 liegt jedoch darin, dass sie in dem Langloch 4 der Knochenplatte 1 lagestabil festgelegt werden kann. Diese Situation ist in Fig. 13 dargestellt. In dieser Stellung greift das Gewinde 13 des Schraubenkopfes 12 in das Gewinde 5 in dem Langloch 4 ein, und die Anlagestruktur 14 liegt formschlüssig an der Haltestruktur 6 an. Durch das Zusammenwirken der Gewinde 5 und 13 wird die Anlagestruktur 14 fest gegen die Haltestruktur 6 gepresst, wobei anzumerken ist, dass die Haltestruktur 6 und das Gewinde 5 ebenso koaxial ausgebildet sind, wie das Gewinde 13 und die Anlagestruktur 14 an dem Schraubenkopf 12. Der Übergang des Schraubenkopfes 12, genauer des Gewindes 13 an dem Schraubenkopf 12 in das Gewinde 5 in dem Langloch 4 wird erleichtert durch den Einstich 7. Hier kann der Anfang des Gewindezuges des Gewindes 13 sicher in das Gewinde 5 überführt werden, so dass er letztlich in dem Gewinde 5 eingreift, ohne zu verkanten.

In einer wie in Fig. 13 gezeigten Position umgreift das Gewinde 5 den Schraubenkopf 12 bzw. das Gewinde 13 an demselben in einer Ebene der Oberfläche 2 der Knochenplatte 1 gesehen um maximal 180°. Gehalten wird die Schraube 11 in ihrer Position aufgrund eines durch die Verkippung der Führungsebene E_{L} gegenüber der Gewindelängsachse A_{G} erzielten Klemmeffekt.

Nur aufgrund der letztgenannten Tatsache ist es möglich, das Langloch 4 tatsächlich durchgehend und über die volle Länge alternativ für die Kompression oder für die winkelstabile Fixierung nutzbar zu gestalten.

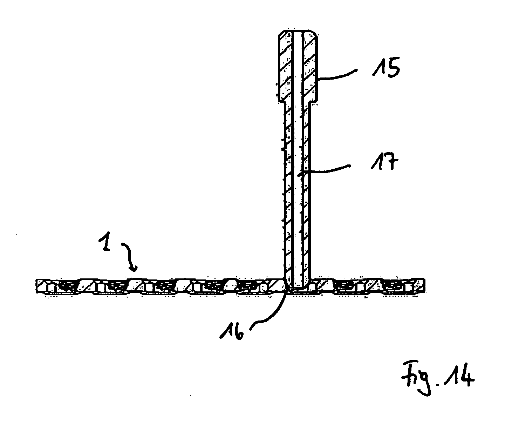

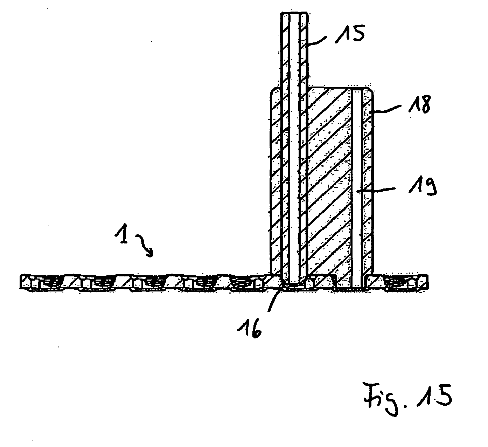

In den Figuren 14 und 15 ist dargestellt, wie für die Operation zum Einbau der Knochenplatte Bohrhülsen mit der Knochenplatte verbunden sind. Die in Figur 14 dargestellte Situation zeigt eine einfache Bohrhülse 15, die mit an ihrem unteren Ende gebildeten sphärischen Gewinde 16 und Anlagestruktur (entsprechend den Strukturen an dem Schraubenkopf 12 der Knochenschraube 11) in das Gewinde 5 und die Haltestruktur 6 auf der Innenseite des Langloches 4 eingeschraubt wird. Die Bohrhülse 15 gibt in ihrem Innern einen Bohrkanal 17 vor, der durch die passgerechte Aufnahme des Gewindes 16 und der Anlagestruktur der Bohrhülse 15 entlang der Achse A_{G} verläuft. Die Bohrhülse 15 dient als Hilfsmittel, um beim Anbohren des mit der Knochenplatte 1 zu versorgenden Knochens sicherzustellen, dass das Bohrloch lotrecht zu der Knochenplatte 1 verläuft und so eine Knochenschraube 11 winkelstabil so in den Knochen geschraubt werden kann, dass ihr Schraubenkopf 12 mit dem Gewinde 13 und der Anlagestruktur 14 problemlos in das Gewinde 5 bzw. die Haltestruktur 15 eingreift.

In Figur 15 ist eine Variante gezeigt, in der auf eine Bohrhülse 15 eine weitere Bohrhülse 18 so aufgesetzt ist, dass der Bohrkanal 19 dieser Bohrhülse 18 exakt parallel zu der Achse A_{G} liegt. Mit dieser Bohrhülse 18 können in den Knochen Schraubenlöcher gebohrt werden, die die oben beschriebene Doppelfunktion einer Knochenschraube 11 erlauben. Die Knochenschraube 11 wird dabei in einem solchen, lotrechten Winkel in den Knochen geschraubt, der es ihr erlaubt, zunächst für eine Kompression entlang der schiefen Ebene E_{L} der Führungsstruktur 8 zu gleiten und anschließend mit dem Gewinde 13 und der Anlagestruktur 14 an ihrem Schraubenkopf 12 sicher und unverkantet in das Gewinde 5 bzw. die Haltestruktur 6 auf der Innenseite des Langloches 4 einzugreifen, um letztlich in ihrem Winkel fixiert zu werden. Dieser Vorgang erlaubt letztlich die gleichzeitige Kompression und Winkelfixierung mit nur einer Schraube in einem Arbeitsgang.

Die gezeigten Bohrhülsen sind rein exemplarisch, und es gibt verschiedene Wege, dieselben Ergebnisse mit anders konstruierten Bohrhülsen zu erzielen.

### Bezugszeichenliste

- 1: Knochenplatte
- 2: Oberseite
- 3: Unterseite
- 4: Langloch
- 5: Gewinde
- 6: Haltestruktur
- 7: Einstich
- 8: Führungsstruktur
- 9: Knochenschraube
- 10: Schraubenkopf
- 11: Knochenschraube
- 12: Schraubenkopf
- 13: Gewinde
- 14: Anlagestruktur
- 15: Bohrhülse
- 16: Gewinde
- 17: Bohrkanal
- 18: Bohrhülse
- 19: Bohrkanal
- A_{G}: Gewindelängsachse
- E_{L}: Führungsebene
- E_{P}: Plattenebene
- α: Winkel
- β: Winkel

## Patentansprüche

1. Knochenplatte (1) mit einer für die Anlage am Knochen ausgebildeten Unterseite (3) und einer vom Knochen abgewandten Oberseite (2) sowie mit einer Mehrzahl von im wesentlichen entlang der Plattenlängsachse vorgesehenen Löchern, durch die zur Verankerung Knochenschrauben (9, 11) mit dem Knochen in Eingriff bringbar sind, wobei wenigstens eines der Löcher als durchgängiges Langloch (4) ausgebildet ist, mit der längeren Achse in Richtung der Plattenlängsachse, wobei in einem Teilbereich des Seitenrandes des Längsloches Gewindezüge (5) vorgesehen sind, die in einer Richtung quer zu der Ebene der Oberseite (2) gesehen nur über einen Teil der Tiefe des Langloches (4) angeordnet sind, **dadurch gekennzeichnet, dass** sich in der Richtung quer zu der Ebene der Oberseite (2) gesehen ober- und/oder unterhalb der Gewindezüge (5) eine glattwandige Haltestruktur (6) zum formschlüssigen Eingriff mit einer entsprechend ausgestalteten Gegenstruktur (14) an einem Schraubenkopf (10, 12) bzw. -hals einer Knochenschraube (9, 11) befindet.

2. Knochenplatte nach Anspruch 1, **dadurch gekennzeichnet, dass** die Gewindezüge (5) in der Ebene der Oberfläche (3) gesehen maximal 180° eines kreisförmigen Umfanges eines mit diesem in Eingriff zu bringenden Schraubenkopfes (10, 12) bzw. -halses einer Knochenschraube (9, 11) umgreift.

3. Knochenplatte nach einem der vorhergehenden Ansprüche, **dadurch gekennzeichnet, dass** die Haltestruktur (6) eine teilsphärische Fläche ist.

4. Knochenplatte nach einem der vorhergehenden Ansprüche, **dadurch gekennzeichnet, dass** die Gewindezüge (5) in einem nahe der Oberseite (2) der Knochenplatte (1) gelegenen Abschnitt angeordnet sind, die Haltestruktur (6) in einem nahe der Unterseite (3) angeordneten Abschnitt.

5. Knochenplatte nach einem der vorhergehenden Ansprüche, **dadurch gekennzeichnet, dass** in einem Übergangsbereich zwischen Gewindezügen (5) und Haltestruktur (6) ein Einstich (7) vorgesehen ist.

6. Knochenplatte nach einem der vorhergehenden Ansprüche, **dadurch gekennzeichnet, dass** das Langloch (4) auf der Oberseite (2) der Knochenplatte (1) eine umlaufende Führungsstruktur (8) zur Führung des Kopfes (10, 12) oder Halses einer Knochenschraube (9, 11) aufweist, wobei die Führungsstruktur (8) eine Führungsebene (E_{L}) definiert und diese Führungsebene (E_{L}) von einer Längsachse des durch die Gewindezüge (5) gebildeten Gewindes um einen von 90° verschiedenen Winkel (β) geschnitten wird.

7. Knochenplatte nach Anspruch 6, **dadurch gekennzeichnet, dass** die Führungsebene (E_{L}), um einen Winkel (α) von größer 0° und kleiner 90° gegenüber der Plattenebene (Ep) geneigt ist.

8. Knochenplatte nach Anspruch 7, **dadurch gekennzeichnet, dass** die Gewindezüge (5) an einer Schmalseite des Langloches (4), vorzugsweise symmetrisch zu dessen längerer Achse, angeordnet ist, wobei in diesem Bereich des Langloches (4) die Führungsstruktur (8) den größten Abstand zu der Oberseite (2) der Knochenplatte (1) aufweist.

9. System bestehen aus einer Knochenplatte (1) nach einem der vorhergehenden Ansprüche und wenigstens einer Knochenschraube (11), wobei die Knochenschraube (11) an einem Schraubenkopf (12) und/oder -hals in einer Richtung entlang ihrer Längsachse nacheinander angeordnet in einem ersten Bereich ein Gewinde (13) und in einem zweiten Bereich eine komplementär zu der Haltestruktur (6) des Langloches (4) der Knochenplatte (1) ausgebildete Anlagestruktur (14) aufweist.

## Claims

1. Bone plate (1) with an underside (3) constructed for engagement on the bone and a bone-remote top side (2), as well as with a plurality of holes provided essentially along the plate longitudinal axis through which by anchoring bone screws (9, 11) can be engaged with the bone, at least one of the holes being constructed as a through elongated hole (4), with the longer axis in the direction of the plate longitudinal axis and in a partial area of the lateral edge of the elongated holes are provided thread grooves (5) which in a direction at right angles to the plane of the top side (2) are provided only over part of the depth of elongated hole (4), **characterized in that** above and/or below the thread grooves (5) in the direction at right angles to the plane of the top side (2) there is a smooth-walled holding structure (6) for positive engagement with a correspondingly designed counterstructure (14) on a screw head (10, 12) or neck of a bone screw (9, 11).

2. Bone plate according to claim 1, **characterized in that**, considered in the plane of surface (3), the thread grooves (4) engage round a maximum of 180° of a circular circumference of a screw head (10, 12) or neck to be brought into engagement therewith.

3. Bone plate according to one of the preceding claims, **characterized in that** the holding structure (6) is a part spherical surface.

4. Bone plate according to one of the preceding claims, **characterized in that** the thread grooves (5) are located in a portion close to the top side (2) of bone plate (1) and the holding structure (6) in a portion located close to the underside (3).

5. Bone plate according to one of the preceding claims, **characterized in that** a recess (7) is provided in a transition area between thread grooves (5) and holding structure (6).

6. Bone plate according to one of the preceding claims, **characterized in that**, on the top side (2) of bone plate (1), elongated hole (4) has a circumferential guide structure (8) for guiding the head (10, 12) or neck of a bone screw (9, 11), the guide structure (8) defining a guide plate (E_{L}), which is intersected by a longitudinal axis of the thread formed by thread grooves (5) by an angle (β) differing from 90°.

7. Bone plate according to claim 6, **characterized in that** the guide plate (E_{L}) is inclined relative to the plate plane (E_{P}) by an angle (α) greater than 0° and smaller than 90°.

8. Bone plate according to claim 7, **characterized in that** the thread grooves (5) are located on a narrow side of the elongated hole (4) and preferably symmetrically to the longer axis thereof and in this area of the elongated hole (4) the guide structure (8) has the maximum spacing from the top side (2) of bone plate (1).

9. System comprising a bone plate (1) according to one of the preceding claims and at least one bone screw (11), which on a screw head (12) and/or neck in a direction along its longitudinal axis are successively provided in a first area a thread (13) and in a second area an engagement structure (14) constructed in complimentary manner to the holding structure (6) of elongated hole (4) of bone plate (1).

## Revendications

1. Plaque d'os (1) comprenant un côté inférieur (3) réalisé pour l'application sur l'os et un côté supérieur (2) opposé à l'os ainsi qu'une pluralité de trous prévus essentiellement le long de l'axe longitudinal de la plaque, par lesquels les vis à os (9, 11) peuvent être amenées en prise avec l'os pour l'ancrage, sachant qu'au moins l'un des trous est réalisé sous forme de trou oblong (4) continu, avec l'axe le plus long en direction de l'axe longitudinal de la plaque, des passages filetés (5) étant prévus dans une zone partielle du bord latéral du trou oblong, lesquels sont disposés, vu dans une direction transversale au plan de la surface (2), seulement sur une partie de la profondeur du trou oblong (4), **caractérisé en ce que**, au-dessus et/ou au-dessous des passages filetés (5), vu dans la direction transversale au plan du côté supérieur (2), une structure de retenue (6) à paroi lisse destinée à l'engagement par complémentarité de formes avec une contre-structure (14) de conception appropriée se trouve sur une tête de vis (10, 12) ou collet d'une vis à os (9, 11).

2. Plaque d'os selon la revendication 1, **caractérisée en ce que** les passages filetés (5) entourent, vu dans le plan de la surface (3), au maximum 180° d'un pourtour circulaire d'une tête de vis (10, 12) ou d'un collet de vis d'une vis à os (9, 11) qui peut être amené(e) en prise avec ce pourtour.

3. Plaque d'os selon l'une quelconque des revendications précédentes, **caractérisée en ce que** la structure de retenue (6) est une surface semi-sphérique.

4. Plaque d'os selon l'une quelconque des revendications précédentes, **caractérisée en ce que** les passages filetés (5) sont disposés dans une partie située à proximité du côté supérieur (2) de la plaque d'os (1), et la structure de retenue (6) dans une partie disposée à proximité du côté inférieur (3).

5. Plaque d'os selon l'une quelconque des revendications précédentes, **caractérisée en ce qu'**une entaille (7) est prévue dans une zone de transition entre les passages filetés (5) et la structure de retenue (6).

6. Plaque d'os selon l'une quelconque des revendications précédentes, **caractérisée en ce que** le trou oblong (4) présente sur le côté supérieur (2) de la plaque d'os (1) une structure de guidage (8) périphérique pour le guidage de la tête (10, 12) ou du collet d'une vis à os (9, 11), la structure de guidage (8) définissant un plan de guidage (E_{L}) et ce plan de guidage (E_{L}) étant coupé par un axe longitudinal du filetage formé par les passages filetés (5) sous un angle (β) différent de 90°.

7. Plaque d'os selon la revendication 6, **caractérisé en ce que** le plan de guidage (E_{L}) est incliné d'un angle (α) supérieur à 0° et inférieur à 90° par rapport au plan de plaque (E_{P}).

8. Plaque d'os selon la revendication 7, **caractérisé en ce que** les passages filetés (5) sont disposés sur un côté étroit du trou oblong (4), de préférence de façon symétrique par rapport à son axe plus long, la structure de guidage (8) présentant la distance maximale au côté supérieur (2) de la plaque d'os (1) dans cette zone du trou oblong (4).

9. Système constitué d'une plaque d'os (1) selon l'une quelconque des revendications précédentes et d'au moins une vis à os (11), la vis à os (11) présentant sur une tête de vis (12) et/ou un collet de vis dans une direction le long de son axe longitudinal, disposé l'un derrière l'autre, dans une première zone un filetage (13) et dans une seconde zone une structure d'appui (14) réalisée de façon complémentaire à la structure de retenue (6) du trou oblong (4) de la plaque d'os (1).
